# EUROPEAN PATENT SPECIFICATION

(11) **EP 0 971 631 B1**
(45) Date of publication and mention of the grant of the patent: **02.01.2002**
(21) Application number: 98913206.3
(22) Date of filing: 27.03.1998
(51) Int. Cl.: A61B 6/10, G21F 3/03

(54) **PERSONAL APPAREL SUPPORT SYSTEM**
TRAGSYSTEM FÜR KLEIDUNGSSTÜCKE
SYSTEME DE SUPPORT POUR APPAREIL PERSONNEL

(30) Priority: 27.03.1997 US 41389 P
(43) Date of publication of application: 19.01.2000
(73) Proprietor: Cook ( Canada) Inc., Stouffville, Ontario L4A 7X5 (CA); Pinkerton, Cass A., Zionsville, IN 46077 (US)
(72) Inventor: PINKERTON, Cass, A., Zionsville, IN 46077 (US); BOBBIE, Bill, A., Unionville, Ontario L3R 3A9 (CA); FUNG, Karl, Ontario, Canada L3Y 8RB (CA); WHITFORD, Wesley, G., Rockwood, Ontario N0B 2K0 (CA); ONDEJKO, Susan, Markham, Ontario L3P 2H7 (CA)
(74) Representative: Johnston, Kenneth Graham
(86) International application number: US9806076
(87) International publication number: WO9842258

(56) References cited:
- DE-A- 2 934 955
- US-A- 3 252 704
- US-A- 3 721 437
- US-A- 4 254 341
- US-A- 4 903 355
- US-A- 5 502 851
- US-A- 5 603 677

## Description

### Technical Field

The present invention relates generally to personal apparel support systems, and more particularly to a personal support system for radiation protective apparel worn by health care providers.

### Background of the Invention

It is known that physicians and other health care providers are frequently required to wear protective apparel, such as lead aprons, in order to protect themselves against stray radiation that is unavoidably emitted from x-ray machines and fluoroscopes. In many cases, physicians and other health care providers are subjected to stray radiation during the entire working day, which when integrated over a long period of time is dangerous to health. To protect themselves against radiation, it is customary for health care providers to wear lead aprons, often for many hours at a time. Over the long term with the constant weight of these lead aprons, back problems develop to such a serious extent that some physicians can no longer practice radiology in suite.

U.S. Patent 4,254,341 to Herr, et al. responds to this problem by providing a radiation protection device in which a lead apron is suspended on a spring from a carriage rolling in a track system that is secured to a ceiling in a room in which the radiation apparatus is located. While the Herr, et al. support system appears to provide an apron wearer with a considerable degree of lateral movement, the device suffers from the considerable disadvantage of requiring a separate device mounted in each room where the physician may be working. Furthermore, Herr, et al. suffers from the disadvantage of having a rather cumbersome means of adjusting their support system to accommodate persons with different heights. In particular, two separate vertically oriented belt-like straps must be adjusted separately in order to position the lead apron at a specific height for a given person. The Herr, et al. adjustment system can therefore result in a left to right imbalance if the straps are not adjusted to have identical lengths. Furthermore, the cumbersome adjustment means of Herr, et al. has the additional disadvantage in that two or more adjustment iterations may be necessary in order to provide adequate support for two different persons using the device.

German Patent No. DE 2934955 A1 to Hänel discloses a suspension apparatus for an X-ray apron which includes a coil spring contained in a weight-balancing housing, the housing being adapted for travel along a rail mounted on the ceiling. The apron is carried on a pullrope connected to a balance drum, the drum being connected in turn to a coil spring. The return tension is adjustable, for example, by turning the axle of the drum. As with any spring-biased device, because the return force of the spring is proportional to the displacement of the spring or the weight which it balances, the weight of the apron of the Hänel device is precisely balanced at only one position. Movement of the wearer from that position either lifts the apron with respect to the wearer, or causes an increased proportion of the weight of the apron to be borne by the wearer. Moreover, adjustment of the Hänel device to any particular user may be less than convenient, since such adjustment must be made at ceiling height.

In contrast, U.S. Patent No. 3,721,437 to Skaricic is directed to a training device for assisting a person in learning to walk. The device is mounted on caster wheels and includes a harness in which the person is suspended, as well as a shock absorber and a tension spring acting to counterbalance the suspended harness. As with the Hänel device, the force applied to support the weight of the person varies with the displacement of the person with respect to the device.

U.S. Patent No. 5,603,677 to Sollo is directed to a similar training device and includes a walker frame capable of gliding movement across a surface, and a harness worn by the person for assistance in lifting the user's body to an upright position. The Sollo device includes a plurality of counterbalance weights on the walker from to provide such assistance, but such assistance is not such as to render the person weightless to either the person's legs or arms.

U.S. Patent No. 3,252,704 to Wilson discloses a lifting and walking jacket for a similar training purpose. The jacket is suspended from an elevated arm or boom carried on base frame having a plurality of caster wheels. The arm is raised by fluid pressure supplied to a fluid cylinder connected to the elevated arm. The arm is lowered by venting fluid through a bleeder valve.

U.S. Patent No. 5,502,851 to Costello is directed to an assisted lifting, standing and walking device which similarly includes a hydraulic jack for adjusting an included lifting mechanism.

U.S. Patent No. 4,903,355 to Hickerson discloses a torso sling for lifting and supporting a physically disabled person. The sling is connected to a hoist or lift whose specific details are not disclosed.

### Summary of the Invention

The foregoing problems are solved and a technical advance is achieved in an illustrative embodiment of a personal apparel support system of the present invention as indicated in claim 1. The system includes a lifting mechanism that includes a retractable cable that extends from a support portion which is positioned above the shoulders of a person. The retractable cable maintains a pre-determined tension that is substantially independent of an amount of the retractable cable that extends below the support portion when an item of apparel is attached to the retractable cable.

In another embodiment of the present invention, a personal apparel support system includes a radiology apron that has a radiation protective material and is sized to be worn by a person. A lifting mechanism includes a cable that extends from a support portion that is positioned above the radiology apron. The cable has at least one counterweight attached to the cable on one side of the support portion. A link has one end attached to the radiology apron and an other end attached to the cable on an opposite side of the support portion. A lateral maneuvering device is attached to the lifting mechanism and includes a base with a plurality of rollers attached thereto. The rollers are provided for making contact with a floor.

In still another embodiment of the present invention, a link is provided for interconnecting a radiology apron having a pair of shoulder portions with a personal apparel support system. The link includes a first V-shaped shoulder bracket having an extension sized to be positioned under part of one of the shoulder portions of the radiology apron. In addition, a second V-shaped shoulder bracket has an extension sized to be positioned under part of the other one of the shoulder portions of the radiology apron.

One object of the present invention is to provide a personal apparel support system that can accommodate a person of virtually any height without adjustments being necessary.

Another object of the present invention is to provide a personal apparel support system that can accommodate different radiology shields having different weights without changing the performance of the device.

Still another object of the present invention is to provide a personal apparel support system that can easily be moved to different locations within a health care institution.

Another object of the present invention is to provide a personal apparel support system that allows a user to choose a desirable amount of support force to suit their individual comfort and needs.

Still another object of the present invention is to provide an improved personal apparel support system.

### Brief Description of the Drawings

FIG. 1 is a side elevational view of a personal apparel support system according to a preferred embodiment of the present invention;
FIG. 2 is a front elevational view of the personal apparel support system of FIG. 1;
FIG. 3 is a sectioned top view of the personal apparel support system of FIG. 1 as viewed along section line 3-3;
FIG. 4 is a partially sectioned side view of the upper portion of the personal apparel support system of FIGs. 1 and 2 as viewed along section line 4-4 of FIG. 2;
FIG. 5 is an isometric view of area 5 of FIG. 1 with portions of the mast removed to reveal inner detail of the lifting mechanism;
FIG. 6 is a partial side sectioned view of the shoulder portion of a radiology apron according to one aspect of the present invention and as viewed along section line 5-5 of FIG. 2; and
FIG. 7 depicts a pictorial view of another aspect of the personal apparel support system of FIG. 1 as including a steering handle.

### Detailed Description

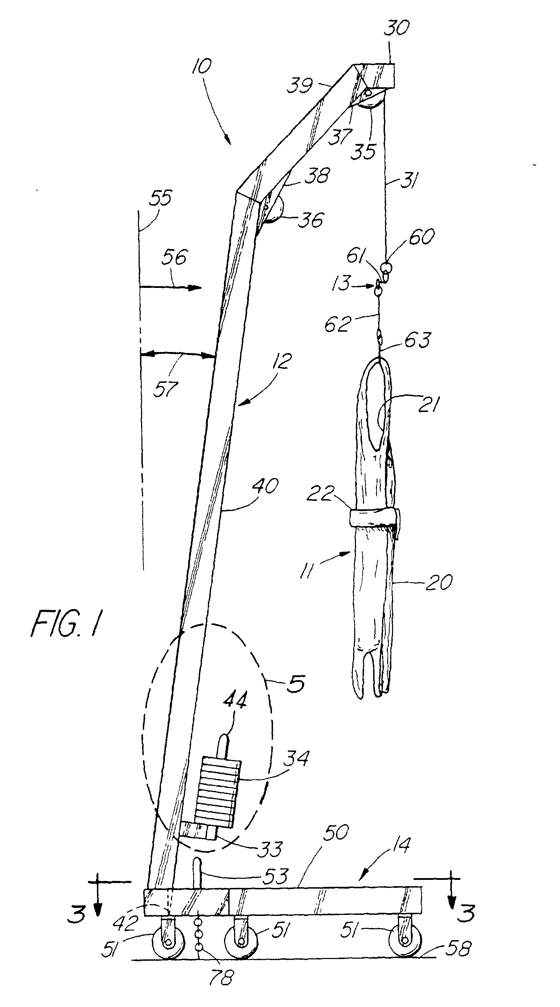

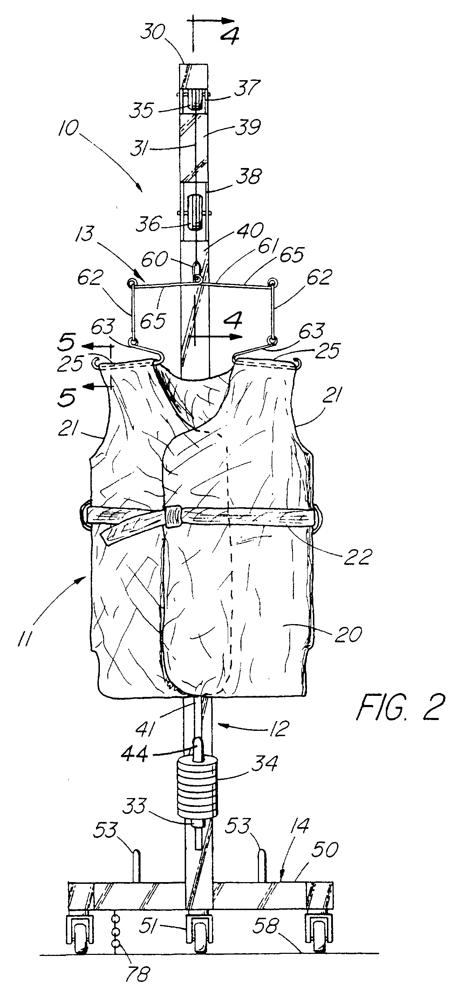

Referring now to FIGs. 1 and 2, a personal apparel support system 10 includes a lifting mechanism 12 attached to and supported by a lateral maneuvering device 14. Support system 10 also includes a link 13 that serves as the means by which an item of personal apparel, such as a radiology shield 11 is supported by lifting mechanism 12. In this case, radiology shield 11 is a radiology apron 20 having a pair of arm openings 21, a belt 22, shoulder portions 25 and an amount of radiation protective material 24, such as lead, enclosed within.

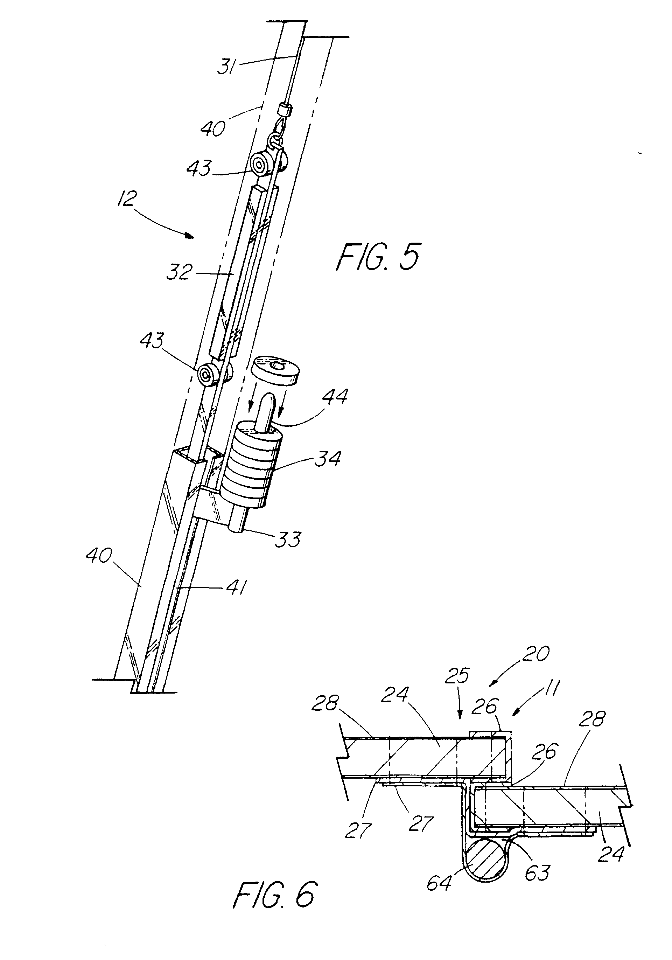

Lifting mechanism 12 includes a retractable cable 31 that extends from a support portion 30, which is positioned at a height above the shoulders of a person wearing radiology apron 20. Support portion 30 of lifting mechanism 12 is attached to an upper mast portion 39, which in turn is attached to a main mast 40. Main mast 40 is preferably tilted away from a vertical line 55 in a direction 56 so that the person wearing radiology apron 20 can move without bumping against mast 40. In the preferred embodiment shown, mast tilt angle 57 is preferably 6.5°, although a range of 0° to 60° is contemplated. Main mast 40 and upper mast portion 39 are preferably manufactured from a commercially available grade of 50.8 mm (2 in) square aluminum tubing. Corner braces 37 and 38 serve as both the means of strengthening the connection between the lifting mechanism components, but also as support means for pulleys 35 and 36. A portion of retractable cable 31 passes over pulleys 35 and 36 and is positioned within the aluminum tubing with an unseen end attached to a sliding counterweight holder 33 (see FIG. 5). By positioning any desired amount of additional counterweights 34 on pin 44 of counterweight holder 33, a pre-determined tension can be maintained in retractable cable 31 that is substantially independent of an amount or the length of the cable that extends below support portion 30 when radiology apron 20 is attached thereto.

End 42 of main mast 40 is attached to and supported by lateral maneuvering device 14. Lateral maneuvering device 14 permits movement of lifting mechanism 12 in a plane substantially perpendicular to gravity. In other words, lateral maneuvering device 14 allows personal apparel support system 10 to be moved about on any horizontal surface, such as a floor 58. Lateral maneuvering device 14 includes a base 50 having a plurality of rollers 51, in this case five, attached thereto.

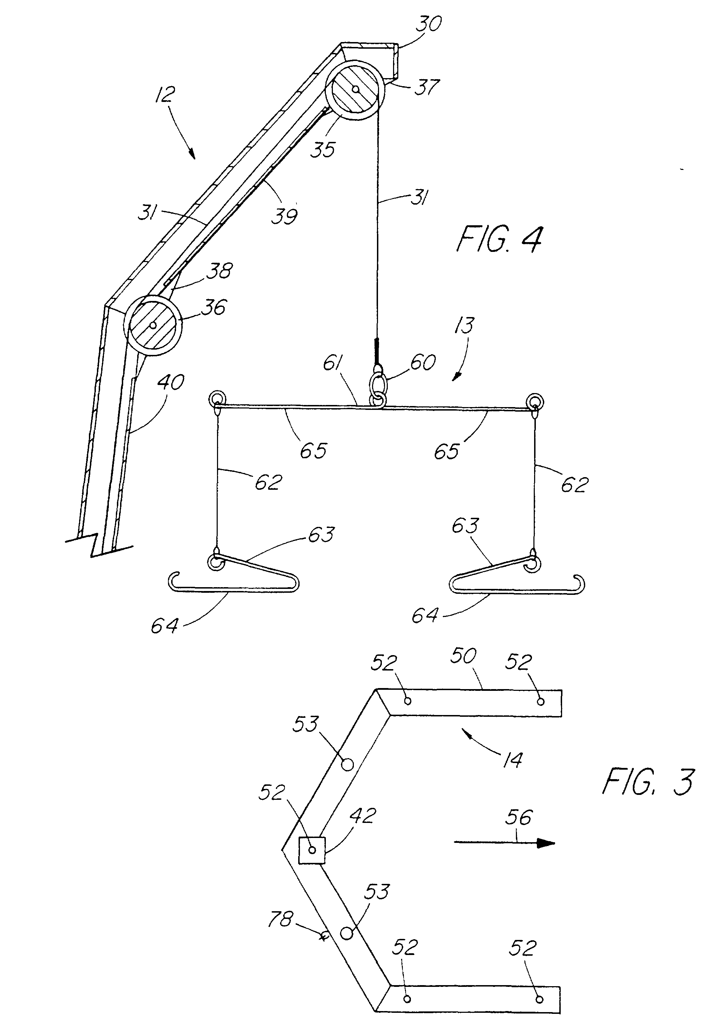

Referring to FIG. 3, base 50 is generally U-shaped with the five rollers attached at corner and end locations 52. Base 50 is preferably formed from welded steel tubing and includes a pair of pins 53 that extend vertically upward for retaining additional counterweights 34. A person using support system 10 typically stands within U-shaped base 50 and faces in direction 56 away from tilted main mast 40. Rollers 51 are preferably, commercially available swivel type caster wheels, although a combination of stationary and swivel type caster wheels are also contemplated.

FIGs. 4 and 5 depict selected views of the embodiment of FIGs. 1 and 2, and, in particular, the working structure of lifting mechanism 12. Retractable cable 31 is preferably, commercially available 3.175 mm (1/8 in) type 302 aircraft cable with loops formed on each end that are positioned on opposite sides of support portion 30. One of the loops attaches to link 13, whereas the opposite loop attaches to a counterweight 32 made up of a plurality of steel plates that are positioned to slide within main mast 40. In order to prevent counterweight 32 from binding when sliding within main mast 40, nylon rollers 43 are attached on each end of counterweight 32. Counterweight holder 33 is attached to counterweight 32 and is slidably received in a guide slot 41 cut into a portion of main mast 40. Counterweight holder 33 includes a guide pin 44 that is received in the apertures of additional counterweights 34 and prevents the same from falling off of holder 33 in case support system 10 is jostled. The intermediate portions of retractable cable 31 pass over grooved pulleys 35 and 36 and allow the cable to move relatively freely in either the direction of link 13 or counterweight holder 33.

On the opposite side of support portion 30 relative to counterweight 32, link 13 is attached to retractable cable 31. Link 13 includes a chain link 60 that interconnects the end loop in retractable cable 31 to an apron hanger 61. Apron hanger 61 is preferably made from 6.350 mm (1/4 in) diameter stainless steel rod and formed to include a central eye portion that is attached to chain link 60 and a pair of outwardly extending arms 65 that each terminate in an eye or hook. A pair of shoulder cables 62, with loops formed on each end, are attached to and hang down from the arms 65 of apron hanger 61. Shoulder cables 62 are preferably made from 1.588 mm (1/16 in) type 302 aircraft cable. Finally, link 13 includes a pair of V-shaped shoulder brackets 63 that are preferably formed from 6.350 mm (1/4 in) diameter stainless steel rod to include eye portions on each end. When attached to shoulder cables 62, shoulder brackets 63 are preferably balanced so that extensions 64 hang in a substantially horizontal orientation. Extensions 64 can be readily positioned under the shoulder portions of virtually any available radiology apron, or sewn into the shoulder portions of a radiology apron as shown in FIGs. 1, 2 and 6 of the preferred embodiment.

Referring now to FIGs. 2 and 6, a radiology apron 20 according to the preferred embodiment includes an outer surface of nylon fabric 28 that encloses a radiation protective material 24, such as lead sheeting, and is bound thereto with appropriate stitching. End joints are further strengthened by stitching in a nylon binding 26 at the ends of radiation protective material 24. Further support is provided on the underside of shoulder portion 25 by stitching a seat belt webbing 27 around each extension 64 of V-shaped shoulder brackets 63.

In the preferred embodiment, an appropriate number of additional counterweights 34 are added to counterweight holder 33 so that the tension in retractable cable 31 is about equal to the weight of radiology apron 20. Additional counterweights can be added to counterweight holder 33 so that there is a net upward force acting through retractable cable 31 on a person wearing radiology apron 20. On the other hand, a particular physician or health care provider may prefer less upward force than the weight of the radiology apron. The present invention can accommodate any of these alternatives. In addition, because the predetermined tension in retractable cable 31 is a direct function of the weight of radiology apron 20 and the amount of counterweight acting on the opposite side of support portion 30, no cumbersome adjustments are necessary in order to adjust support system 10 to accommodate persons having different heights, other than having to provide more or less cable.

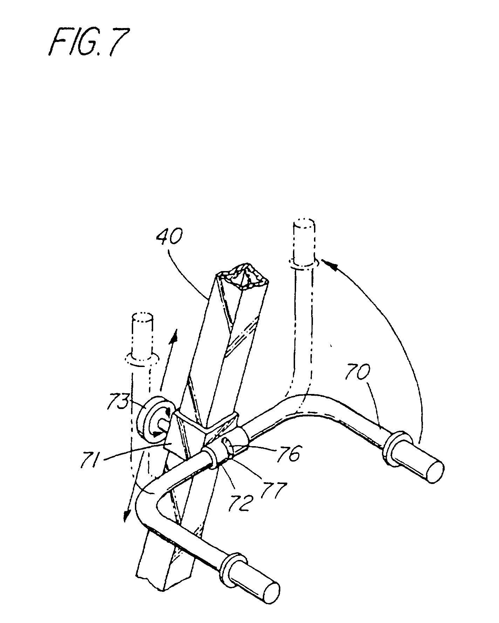

FIG. 7 depicts a pictorial view of another aspect of the personal apparel support system of FIG. 1 as including a steering handle 70. The steering handle is disposed on main mast 40 with the aid of slidable bracket 71 and threaded knob 73 for fixing the vertical position of the steering handle along main mast 40. This slidable positioning conveniently permits the physician to adjust the vertical height of the handle so as to facilitate easy movement and directional control of the personal apparel support system 10. Slidable bracket 71 also includes a swivel mount 72 with travel limiting slot 76 formed therein. Pin 77 extends from the middle portion of the handle and in the travel limiting slot to facilitate movement of the handle from a horizontal position to a vertical position as depicted by the phantom lines. When in the vertical position, the steering handle does not interfere with the arm movement of the physician or health care provider.

Those skilled in the art will appreciate that the above description is intended for illustrative purposes only, and is not intended to limit the scope of the present invention in any way.

Furthermore, while the lateral maneuvering device of the present invention has been illustrated as a base having rollers in contact with the floor, those skilled in the art will appreciate that the present invention could be mounted on a moveable track to the ceiling of a particular room without altering the underlying concepts of the present invention. Furthermore, other enhancements, such as friction brakes, etc. may be incorporated into the personal apparel support system to further enhance the comfort of the system to some users. By way of another example, static electricity or charge grounding chain 78 can be attached to base 50 to eliminate static charge buildup. In any event, the scope of the present invention should be determined in terms of the claims as set forth below.

## Claims

1. A personal apparel support system (10) including a lifting mechanism (12) including a retractable cable (31) that extends from a support portion (30) and varies in length to accommodate a person of virtually any height and to which an item (11) of personal apparel is attachable thereto; **characterized in that** the lifting mechanism (12) includes at least one counterweight (32), which is attachable to the retractable cable (31) on one side of the support portion (30), for maintaining in the retractable cable (31) a predetermined tension that is substantially independent of the amount or length of said retractable cable (31) that extends below and on an opposite side of said support portion (30) when the item (11) of personal apparel is attached to the retractable cable (31), whereby the predetermined tension in the retractable cable (31) would be adequate to counterweigh at least part of the weight of the item (11) of personal apparel.

2. The personal apparel support system (10) of claim 1 **characterized in that** the support system (10) further includes a lateral maneuvering device (14) that is attached to and supports said lifting mechanism (12), and **in that** said lateral maneuvering device (14) permits movement of said support portion (30) of said lifting mechanism (12).

3. The personal apparel support system (10) of claim 2 **characterized in that** said lateral maneuvering device (14) includes a base (50) with a plurality of rollers (51) attached thereto.

4. The personal apparel support system (10) of claim 3 **characterized in that** said lifting mechanism (12) includes a mast (40) with one end (42) attached to said base (50) and an upper portion (39) attached to said support portion (30).

5. The personal apparel support system (10) of claim 4 **characterized in that** said lifting mechanism (12) further includes a counterweight holder (33) attached to said retractable cable (31) on the one side of said support portion (30), and **in that** said counterweight holder (33) is slidably mounted on said mast (40).

6. The personal apparel support system (10) of claim 5 **characterized in that** the support system (10) further includes a link (13) attached to said retractable cable (31) on the opposite side of said support portion (30) relative to said counterweight holder (33); and **in that** the item (11) of personal apparel comprises a radiology shield (11) that is attached to said link (13), includes a radiation protective material (24), and is sized to substantially cover the chest of a person.

7. The personal apparel support system (10) of claim 6 **characterized in that** said radiology shield (11) is a radiology apron (20) with a pair of shoulder portions (25); and **in that** said link (13) includes at least one shoulder bracket (63) with extensions (64) positioned under part of said shoulder portions (25) of said radiology apron (20).

8. The personal apparel support system (10) of claim 7 **characterized in that** said link (13) further includes an apron hanger (61) and a pair of shoulder cables (62), and **in that** each of said shoulder cables (62) has one end attached to said apron hanger (61) and an opposite end attached to said at least one shoulder bracket (63).

9. The personal apparel support system (10) of claim 5 **characterized in that** said base (50) of said lateral maneuvering device (14) includes a counterweight retaining pin (53).

10. The personal apparel support system (10) of claim 2 **characterized in that** the support system (10) further includes a steering handle (70) disposed on said lifting mechanism (12).

11. The personal apparel support system (10) of claim 1 **characterized in that** said item (11) of personal apparel has a weight and **in that** said at least one counterweight (32) is about equal to said weight.

12. The personal apparel support system (10) of claim 6 **characterized in that** the link (13) includes a first V-shaped shoulder bracket (63) having an extension (64) that is sized for positioning under a first shoulder portion (25) of said radiology shield (11) and further includes a second V-shaped shoulder bracket (63) having an extension (64) that is sized for positioning under another shoulder portion (25) of said radiology shield.

13. The personal apron support system (10) of claim13 **characterized in that** the link (13) further includes a shield hanger (61) with a pair of arms (65); and **in that** each of said first V-shaped shoulder bracket (63) and said second V-shaped shoulder bracket (63) is attached to a different one of said pair of arms (65).

## Patentansprüche

1. Stützsystem (10) für Personenschutzkleidung, enthaltend einen Hebemechanismus (12) mit einem einziehbaren Kabel (31), das sich von einem Stützteil (30) aus erstreckt, zur Anpassung an Personen praktisch jeder beliebigen Körpergröße unterschiedlich lang ist und an dem ein Teil (11) der Personenschutzkleidung befestigt werden kann; **dadurch gekennzeichnet, dass** der Hebemechanismus (12) mindestens ein am einziehbaren Kabel (31) an einer Seite des Stützteils (30) befestigbares Gegengewicht (32) zum Aufrechterhalten einer vorbestimmten, im wesentlichen von der Menge oder Länge des einziehbaren Kabels (31) unabhängigen Spannung enthält in dem einziehbaren Kabel (31) wobei sich das gegengewicht, unter und auf einer gegenüberliegenden Seite des Stützteils (30) erstreckt, wenn der Teil (11) der Personenschutzkleidung am einziehbaren Kabel (31) befestigt ist, wodurch die vorbestimmte Spannung im einziehbaren Kabel (31) ausreichen würde, um zumindest einen Teil des Gewichts des Teils (11) der Personenschutzkleidung auszugleichen.

2. Stützsystem (10) für Personenschutzkleidung nach Anspruch 1, **dadurch gekennzeichnet, dass** das Stützsystem (10) ferner eine seitliche Manövriereinrichtung (14) enthält, die an dem Hebemechanismus (12) befestigt ist und diesen stützt, und dass diese seitliche Manövriereinrichtung (14) die Bewegung des Stützteils (30) des Hebemechanismus (12) gestattet.

3. Stützsystem (10) für Personenschutzkleidung nach Anspruch 2, **dadurch gekennzeichnet, dass** die seitliche Manövriereinrichtung (14) einen Sockel (50) mit einer Vielzahl daran befestigter Rollen (51) enthält.

4. Stützsystem (10) für Personenschutzkleidung nach Anspruch 3, **dadurch gekennzeichnet, dass** der Hebemechanismus (12) einen Mast (40), dessen eines Ende (42) an dem Sockel (50) und dessen Oberteil (39) an dem Stützteil (30) befestigt ist, enthält.

5. Stützsystem (10) für Personenschutzkleidung nach Anspruch 4, **dadurch gekennzeichnet, dass** der Hebemechanismus (12) weiterhin einen am einziehbaren Kabel (31) an einer Seite des Stützteils (30) befestigten Gegengewichthalter (33) enthält und dass der Gegengewichthalter (33) gleitend auf dem Mast (40) angeordnet ist.

6. Stützsystem (10) für Personenschutzkleidung nach Anspruch 5, **dadurch gekennzeichnet, dass** das Stützsystem (10) weiterhin eine am einziehbaren Kabel (31) auf der jenem Stützteil (30) gegenüberliegenden Seite relativ zum Gegengewichthalter (33) befestigte Verbindung (13) enthält; und dass der Teil (11) der Personenschutzkleidung eine Röntgenabschirmung (11) enthält, die an der Verbindung (13) befestigt ist, ein Strahlenschutzmaterial (24) umfasst und deren Größe so bemessen ist, dass sie die Brust einer Person im wesentlichen bedeckt.

7. Stützsystem (10) für Personenschutzkleidung nach Anspruch 6, **dadurch gekennzeichnet, dass** die Röntgenabschirmung (11) eine Bleischürze (20) mit einem Paar Schulterteile (25) ist, und dass die Verbindung (13) mindestens einen Schulterbügel (63) mit unter einem Teil der Schulterteile (25) der Bleischürze (20) angeordneten Verlängerungen (64) enthält.

8. Stützsystem (10) für Personenschutzkleidung nach Anspruch 7, **dadurch gekennzeichnet, dass** die Verbindung (13) ferner einen Schürzenaufhänger (61) und ein Paar Schulterkabel (62) enthält, und dass jeweils ein Ende jedes dieser Schulterkabel (62) am Schürzenaufhänger (61) befestigt ist und ein gegenüberliegendes Ende an dem mindestens einen Schulterbügel (63) befestigt ist.

9. Stützsystem (10) für Personenschutzkleidung nach Anspruch 5, **dadurch gekennzeichnet, dass** der Sockel (50) der seitlichen Manövriereinrichtung (14) einen Gegengewicht-Haltestift (53) enthält.

10. Stützsystem (10) für Personenschutzkleidung nach Anspruch 2, **dadurch gekennzeichnet, dass** das Stützsystem (10) ferner einen am Hebemechanismus (12) angeordneten Steuergriff (70) enthält.

11. Stützsystem (10) für Personenschutzkleidung nach Anspruch 1, **dadurch gekennzeichnet, dass** der Teil (11) der Personenschutzkleidung ein Gewicht aufweist und dass mindestens ein Gegengewicht (32) ungefähr diesem Gewicht entspricht.

12. Stützsystem (10) für Personenschutzkleidung nach Anspruch 6, **dadurch gekennzeichnet, dass** die Verbindung (13) einen ersten V-förmigen Schulterbügel (63) mit einer Verlängerung (64), deren Größe so bemessen ist, dass sie unter einem ersten Schulterteil (25) der Röntgenabschirmung (11) angeordnet werden kann, sowie ferner einen zweiten V-förmigen Schulterbügel (63) mit einer Verlängerung (64), deren Größe so bemessen ist, dass sie unter einem anderen Schulterteil (25) der Röntgenabschirmung angeordnet werden kann, enthält.

13. Stützsystem (10) für Personenschutzkleidung nach Anspruch 13, **dadurch gekennzeichnet, dass** die Verbindung (13) ferner einen Abschirmungsaufhänger (61) mit einem Paar Arme (65) enthält, und dass der erste V-förmige Schulterbügel (63) und der zweite V-förmige Schulterbügel (63) jeweils an einem anderen Arm des Paars Arme (65) befestigt sind.

## Revendications

1. Système de support (10) pour appareil personnel comprenant un mécanisme de levage (12) comportant un câble rétractable (31) qui s'étend à partir d'une portion de support (30) et dont la longueur varie de manière à s'adapter à une personne de virtuellement n'importe quelle taille, et auquel il est possible d'attacher un élément (11) d'un appareil personnel; **caractérisé en ce que** le mécanisme de levage (12) comprend au moins un contrepoids (32) pouvant être attaché au câble rétractable (31) d'un côté de la portion de support (30), pour maintenir dans le câble rétractable (31) une tension prédéterminée qui est substantiellement indépendante de la quantité ou de la longueur dudit câble rétractable (31) qui s'étend en dessous et sur un côté opposé de ladite portion de support (30) lorsque l'élément (11) d'un appareil personnel est attaché au câble rétractable (31), la tension prédéterminée dans le câble rétractable (31) étant de ce fait apte à contrebalancer au moins une partie du poids de l'élément (11) d'un appareil personnel.

2. Système de support (10) pour appareil personnel conformément à la revendication 1, **caractérisé en ce que** le système de support (10) comprend en outre un dispositif de manoeuvre latérale (14) qui est attaché à et qui supporte ledit mécanisme de levage (12), et **en ce que** ledit dispositif de manoeuvre latérale (14) permet un mouvement de ladite portion de support (30) dudit mécanisme de levage (12).

3. Système de support (10) pour appareil personnel conformément à la revendication 2, **caractérisé en ce que** ledit dispositif de manoeuvre latérale (14) comprend une base (50) à laquelle sont attachées une pluralité de roulettes (51).

4. Système de support pour appareil personnel (10) conformément à la revendication 3, **caractérisé en ce que** ledit mécanisme de levage (12) comprend un mât (40) avec une extrémité (42) attachée à ladite base (50) et une portion supérieure (39) attachée à ladite portion de support (30).

5. Système de support (10) pour appareil personnel conformément à la revendication 4, **caractérisé en ce que** ledit mécanisme de levage (12) comprend un outre un support de contrepoids (33) attaché audit câble rétractable (31) sur le premier côté de ladite portion de support (30), et **en ce que** ledit support de contrepoids (33) est monté de façon coulissante sur ledit mât (40).

6. Système de support (10) pour appareil personnel conformément à la revendication 5, **caractérisé en ce que** le système de support (10) comprend en outre une suspension (13) attachée audit câble rétractable (31) sur le côté opposé de ladite portion de support (30) par rapport audit support de contrepoids (33); et **en ce que** l'élément (11) d'un appareil personnel comprend un écran de radiologie (11) qui est attaché à ladite suspension (13), comprend une matière de protection contre les radiations (24), et dont la taille permet de substantiellement couvrir la poitrine d'une personne.

7. Système de support (10) pour appareil personnel conformément à la revendication 6, **caractérisé en ce que** ledit écran de radiologie (11) est un tablier de radiologie (20) avec une paire de portions d'épaule (25); et **en ce que** ladite suspension (13) comprend au moins un cintre d'épaule (63) avec des extensions (64) positionnées en dessous d'une partie desdites portions d'épaule (25) dudit tablier de radiologie (20).

8. Système de support (10) pour appareil personnel conformément à la revendication 7, **caractérisé en ce que** ladite suspension (13) comprend en outre une potence de tablier (61) et une paire de câbles d'épaule (62), et **en ce que** chacun desdits câbles d'épaule (62) a une extrémité attachée à ladite potence de tablier (61) et une extrémité opposée attachée audit au moins un cintre d'épaule (63).

9. Système de support (10) pour appareil personnel conformément à la revendication 5, **caractérisé en ce que** ladite base (50) dudit dispositif de manoeuvre latérale (14) comprend une cheville de retenue de contrepoids (53).

10. Système de support (10) pour appareil personnel conformément à la revendication 2, **caractérisé en ce que** le système de support (10) comprend en outre une poignée de commande (70) disposée sur ledit mécanisme de levage (12).

11. Système de support (10) pour appareil personnel conformément à la revendication 1, **caractérisé en ce que** ledit élément (11) d'un appareil personnel pèse un certain poids et **en ce que** ledit au moins un contrepoids (32) est approximativement égal audit poids.

12. Système de support (10) pour appareil personnel conformément à la revendication 6, **caractérisé en ce que** la suspension (13) comprend un premier cintre d'épaule en forme de V (63) présentant une extension (64) qui est dimensionnée de manière à se positionner en dessous d'une première portion d'épaule (25) dudit écran de radiologie (11), et comprend en outre un deuxième cintre d'épaule en forme de V (63) présentant une extension (64) qui est dimensionnée de manière à se positionner en dessous dune autre portion d'épaule (25) dudit écran de radiologie.

13. Système de support (10) pour tablier personnel conformément à la revendication 13, **caractérisé en ce que** la suspension (13) comprend en outre une potence d'écran (61) avec une paire de bras (65); et **en ce que** tant ledit premier cintre d'épaule en forme de V (63) que ledit deuxième cintre d'épaule en forme de V (63) est attaché à un bras différent de ladite paire de bras (65).
